# EUROPEAN PATENT SPECIFICATION

(11) **EP 4 373 495 B1**
(45) Date of publication and mention of the grant of the patent: **19.11.2025**
(21) Application number: 22754382.4
(22) Date of filing: 20.07.2022
(51) Int. Cl.: A61K 31/573, A61K 31/661, A61P 11/06, A61P 29/00

(54) **BUDESONIDE 21-PHOSPHATE SODIUM SALT FOR USE AS ANTI-INFLAMMATORY OR ANTI-ASTHMATIC AGENT**
BUDESONID-21-PHOSPHAT-NATRIUMSALZ ZUR VERWENDUNG ALS ENTZÜNDUNGSHEMMENDES ODER ANTIASTHMATISCHES MITTEL
SEL DE SODIUM DE BUDÉSONIDE 21-PHOSPHATE DESTINÉ À ÊTRE UTILISÉ COMME AGENT ANTI-INFLAMMATOIRE OU ANTI-ASTHMATIQUE

(30) Priority: 21.07.2021 IT 202100019355
(43) Date of publication of application: 29.05.2024
(73) Proprietor: Genetic S.p.A., 84083 Castel San Giorgio (SA) (IT)
(72) Inventor: CALIENDO, Giuseppe, 80034 Marigliano (NA) (IT); CIRINO, Giuseppe, 80123 Napoli (NA) (IT); FIORINO, Ferdinando, 82100 Benevento (BN) (IT); FRECENTESE, Francesco, 81031 Aversa (CE) (IT); MUSCARA', Marcelo Nicolas, 13025-900 Campinas (BR); PERISSUTTI, Elisa, 86079 Venafro (IS) (IT); PETTI, Antonio, 84091 Battipaglia (SA) (IT); ROVIEZZO, Fiorentina, 82013 Bonea (BN) (IT); SANTAGADA, Vincenzo, 80127 Napoli (NA) (IT); SEVERINO, Beatrice, 80018 Mugnano di Napoli (NA) (IT); MAGLI, Elisa, 80055 Portici (NA) (IT)
(74) Representative: Dragotti & Associati S.P.A.
(86) International application number: PCT/EP2022/070349
(87) International publication number: WO 2023/001890

(56) References cited:
- WO-A1-2021/191366
- WOUTER HOFKENS ET AL: "Safety of glucocorticoids can be improved by lower yet still effective dosages of liposomal steroid formulations in murine antigen-induced arthritis: Comparison of prednisolone with budesonide", INTERNATIONAL JOURNAL OF PHARMACEUTICS, ELSEVIER, NL, vol. 416, no. 2, 26 February 2011 (2011-02-26), pages 493 - 498, XP028265151, ISSN: 0378-5173, [retrieved on 20110304], DOI: 10.1016/J.IJPHARM.2011.02.062
- BANCIU M ET AL: "Liposomal glucocorticoids as tumor-targeted anti-angiogenic nanomedicine in B16 melanoma-bearing mice", JOURNAL OF STEROID BIOCHEMISTRY & MOLECULAR BIOLOGY, ELSEVIER SCIENCE LTD., OXFORD, GB, vol. 111, no. 1-2, 1 July 2008 (2008-07-01), pages 101 - 110, XP023314537, ISSN: 0960-0760, [retrieved on 20080707], DOI: 10.1016/J.JSBMB.2008.05.004
- JEFFREY C. KERN ET AL: "Novel Phosphate Modified Cathepsin B Linkers: Improving Aqueous Solubility and Enhancing Payload Scope of ADCs", BIOCONJUGATE CHEMISTRY, vol. 27, no. 9, 28 July 2016 (2016-07-28), US, pages 2081 - 2088, XP055557206, ISSN: 1043-1802, DOI: 10.1021/acs.bioconjchem.6b00337

## Description

The present invention relates to budesonide 21-phosphate sodium salt for use as anti-inflammatory agent or as anti-asthmatic agent. The invention further relates to pharmaceutical compositions containing the same.

### BACKGROUND OF THE INVENTION

Budesonide (Bud) (chemical name 11β,21-dihydroxy-16α,17α-(butylidenebis(oxy))pregna-1,4-diene-3,20-dione), is a glucocorticoid steroid for the treatment of asthma, chronic obstructive pulmonary disease (COPD), noninfectious rhinitis and Crohn disease, represented by Formula I.

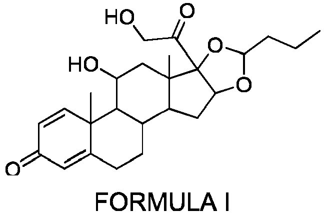

Budesonide has a logP of 3.2 and results practically insoluble in water (28 µg/mL) [Journal of Chemical and Engineering Data (2010), vol. 55, no. 1, pp. 578-582] at physiological pH in the intestinal region. It belongs to inhaled corticosteroids (ICS), a class of compounds that represents, by far, the most effective therapeutic tool used in the treatment of asthma, able to suppress and activate many genes relevant to elicit inflammation in asthmatic airways, even in very low doses.

WO2021/191366 discloses budesonide 21-phosphate for use as anti-inflammatory agent or as anti-asthmatic agent. INTERNATIONAL JOURNAL OF PHARMACEUTICS, vol. 416, no. 2, pp 493-8 shows liposamal budesonide-phosphate to have anti-inflammatory (anti-arthritic) activity.

Budesonide 21-phosphate sodium salt (hereinafter also referred to as Bud-21 P-Na₂) (Formula II) has been used in some studies for the preparation of liposomal glucocorticoids studied as antitumor agents [Journal of Steroid Biochemistry & Molecular Biology 111 (2008) 101-110; Journal of Controlled Release 127 (2008) 131-136].

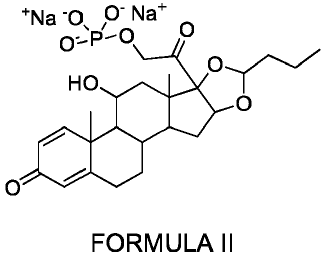

In the present invention compound of Formula II is described as a more water-soluble compound with anti-inflammatory and anti-asthmatic properties greater than budesonide.

### DEFINITIONS

Unless otherwise defined, all terms of art, notations and other scientific terminology used herein are intended to have the meanings commonly understood by those of skill in the art to which this disclosure pertains. In some cases, terms with commonly understood meanings are defined herein for clarity and/or for ready reference; thus, the inclusion of such definitions herein should not be construed to represent a substantial difference over what is generally understood in the art.

The term **"physiologically acceptable excipient"** herein refers to a substance devoid of any pharmacological effect of its own and which does not produce adverse reactions when administered to a mammal, preferably a human. Physiologically acceptable excipients are well known in the art and are disclosed, for instance in the Handbook of Pharmaceutical Excipients, sixth edition 2009, herein incorporated by reference.

The term **"Budesonide 21-phosphate sodium salt"** herein refers to the disodium salt of Budesonide 21-phosphate.

The term **"simultaneous, separate or sequential administration"** herein refers to administration of the first and second compound at the same time or in such a manner that the two compound act in the patient's body at the same time or administration of one compound after the other compound in such a manner to provide a therapeutic effect. In some embodiments the compounds are taken with a meal. In other embodiments, the compounds are taken after a meal, such as 30 minutes or 60 minutes after a meal. In some embodiments, one compound is administered to a patient for a time period followed by administration of the other compound.

As used herein, a **"therapeutically effective amount"** refers to an amount sufficient to achieve treatment or prevention of the disease. Determination of the effective amounts is well within the capability of those skilled in the art based upon the achievement of a desired effect. An effective amount will depend on factors including, but not limited to, the weight of a subject and/or the degree of the disease or unwanted condition from which a subject suffers.

The terms **"treatment"** and **"prevention"** as used herein refer to the eradication/amelioration or prevention/delay in onset, respectively, of the disorder being treated or of one or more of the symptoms associated thereof, notwithstanding the fact that the patient may still be afflicted with the underlying disorder.

The terms "approximately" and "about" herein refer to the range of the experimental error, which may occur in a measurement.

The terms "comprising", "having", "including" and "containing" are to be construed open-ended terms (i. e. meaning "including, but not limited to") and are to be considered as providing support also for terms as "consist essentially of", "consisting essentially of", "consist of" or "consisting of".

The terms "consist essentially of", "consisting essentially of" are to be construed as semi-closed terms, meaning that no other ingredients which materially affects the basic and novel characteristics of the invention are included (optional excipients may thus be included).

The terms "consists of", "consisting of" are to be construed as closed terms.

### SUMMARY OF THE INVENTION

Budesonide is virtually insoluble in water while it results readily soluble in alcohols. For this reason, hydroalcoholic solutions are usually prepared dissolving an adequate amount of active substance in solubilizers such as water-soluble alcohols. However, the so prepared solutions have low stability because large amounts of budesonide are decomposed within a short time. Moreover, budesonide formulations have been prepared until now in the form of aqueous suspensions in which the solid phase tends in time to deposit onto the bottom of the container, thus requiring chemical additives or vigorous stirring. These are the reasons that make budesonide not suitable to be delivered by an electric nebulizer.

Budesonide 21-phosphate sodium salt (hereinafter also referred to as Bud-21 P-Na₂) (Formula II) has been used in some studies for the preparation of liposomal glucocorticoids studied as antitumor agents.

Journal of Controlled Release 127 (2008) 131-136 discloses budesonide disodium phosphate (BUP) encapsulated in long-circulating liposomes (LCL) had the highest antitumor activity which is likely due to the much higher potency of BUP encapsulated in LCL versus the other three GC types (prednisolone, dexamethasone, and methylprednisolone disodium phosphate). The high potency of LCL-BUP confers the risk for occurrence of strong side effects.

Journal of Steroid Biochemistry & Molecular Biology 111 (2008) 101-110 discloses that among the four LCL-GC types studied, namely: budesonide sodium phosphate (BUP), dexamethasone disodium phosphate (DXP), methylprednisolone disodium phosphate (MPLP), and prednisolone phosphate (PLP), LCL-BUP show the highest antitumor activity, which is likely related to the strong potency of this GC to reduce the production of pro-angiogenic and pro-inflammatory factors in tumors.

In the present invention Bud-21 P-Na₂ is described as an anti-inflammatory and anti-asthmatic agent having a greater activity than budesonide.

The present inventors have surprisingly found that Bud-21 P-Na₂ is useful in the prevention and/or treatment of acute or chronic inflammatory diseases, preferably respiratory inflammatory pathologies, obstructive pathologies, and allergen-induced airway dysfunctions.

Accordingly, a first object of the present invention is Bud-21 P-Na₂ for use as anti-inflammatory agent or as anti-asthmatic agent.

The second object of the present invention is a pharmaceutical composition comprising Bud-21 P-Na₂ in combination with at least one physiologically acceptable excipient, for use as anti-inflammatory agent or as anti-asthmatic agent.

According to one preferred embodiment, Bud-21 P-Na₂ and its pharmaceutical composition as defined above, are useful in the prevention and/or treatment of acute or chronic inflammatory diseases, preferably respiratory inflammatory pathologies, obstructive pathologies, allergen-induced airway dysfunctions, such as asthma, COPD and pulmonary fibrosis (e.g. cystic fibrosis), skin inflammatory diseases, such as psoriasis, rheumatoid arthritis, lupus, arthralgia, inflammatory neurodegenerative diseases, such as multiple sclerosis, acute or chronic neuritis.

The third object of the present invention is a product or a kit for use as defined above, comprising budesonide 21-phosphate sodium salt or its pharmaceutical composition and one or more pharmaceutically active compounds (preferably, antibiotics) for simultaneous, separate or sequential use.

### DESCRIPTION OF THE FIGURES

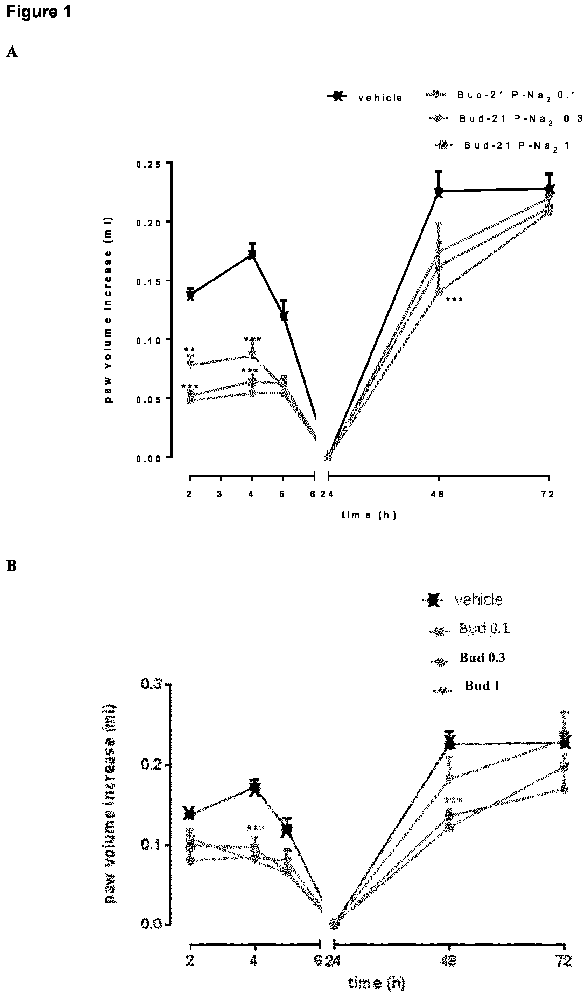

**Figure 1** shows the dose related effect of budesonide 21-phosphate sodium salt and budesonide on carrageenan-induced paw edema.

Edema was assessed after the sub-plantar injection of carrageenan. The animals were pre-treated (i.p.) with corticosteroids 60 min before edema induction. **Panel A** shows the results obtained after treatment with Bud-21 P-Na₂ at doses of 0.1, 0.3 and 1mg/kg. **Panel B** shows the results obtained after treatment with Bud at doses of 0.1, 0.3 and 1mg/kg. *P<0.05, **P<0.01 and ***P<0.001 vs. vehicle-treated group, as analyzed by one-way ANOVA followed by the Fisher's test.

**Figure 2** shows the comparative effects of budesonide 21-phosphate sodium salt and budesonide at dose of 0.3 mg/kg on carrageenan-induced paw edema.

Edema was assessed after the sub-plantar injection of carrageenan. The animals were pre-treated (i.p.) with corticosteroids 60 min before edema induction.

**Figure 3** shows the comparative effects of budesonide 21-phosphate sodium salt and budesonide on cell infiltration in air pouch model.

Cell infiltration has been quantified at 24 hours after the injection of 10 µg of OVA into the dorsal air pouch of sensitized mice; the cell infiltrate is mainly made up of the polymorphonuclear population (PMN) and a significant proportion of eosinophils. The compounds have been administered 60 minutes before OVA injection into air pouch. *P<0.05 vs vehicle.

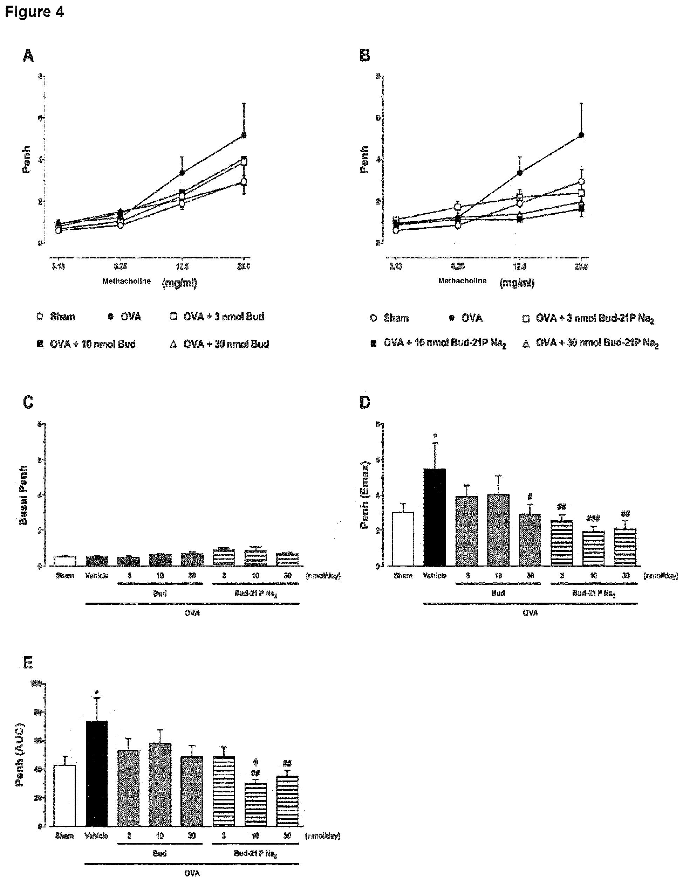

**Figure 4** shows the comparative effects of budesonide and its 21-phosphate sodium salt on bronchoconstriction in an experimental model of allergic asthma.

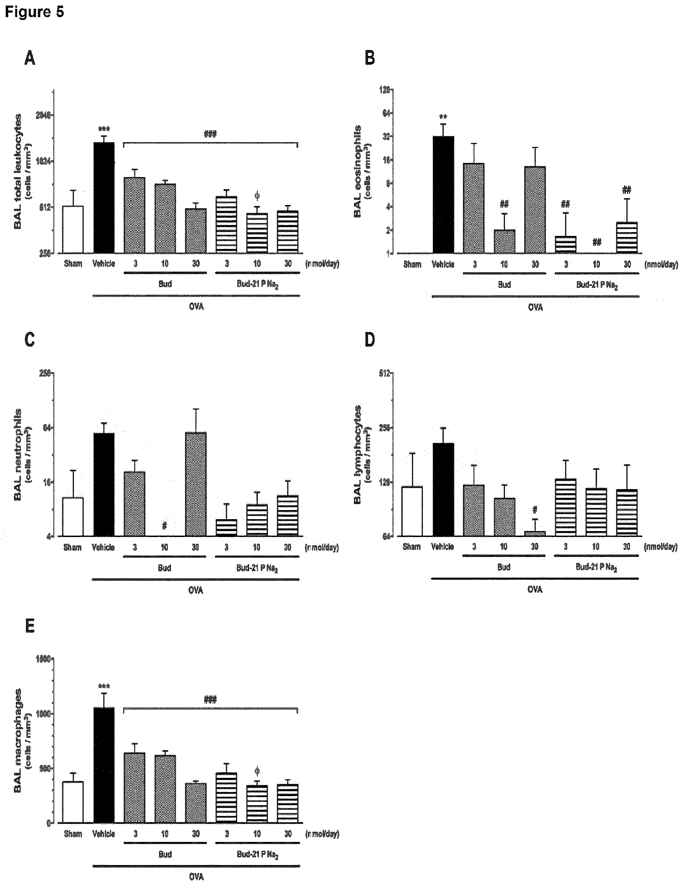

The enhanced pause response (Penh) was measured after exposure of the unrestrained animals to inhaled methacholine at increasing concentrations *(panels A and B)* after the daily administration of the compounds during 4 weeks (following the first OVA-challenge). Each data point represents meantSEM for n=5-6 animals/group. *Panel C* Basal Penh measurement (i.e., no methacholine exposure); *panel D:* peak Penh responses (Eₘₐₓ); *panel E:* areas under the Penh vs. MCh dose curves (AUC). Data were analyzed by one-way ANOVA followed by the Fisher's LSD test for multiple mean comparisons. *P<0.05 *vs.* Sham; ^{#}P<0.05, ^{##}P<0.01 and ^{###}P<0.001 vs. untreated OVA group; ^{ϕ}P<0.05 *vs.* the respective Bud equimolar dose. **Figure 5** shows the comparative effects of budesonide and its 21-phosphate sodium salt on total and differential leukocytes counts in BAL fluid in an experimental model of allergic asthma.

BAL fluids were collected from untreated Sham and OVA-induced allergic animals after the daily administration of the compounds for 4 weeks (following the first OVA-challenge). Bars represent meantSEM for n=5-6 animals/group. *Panel A:* total BAL leukocytes; *panel B:* BAL eosinophils; *panel C:* BAL neutrophils; *panel D:* BAL lymphocytes; *panel E:* BAL macrophages. Data were analyzed by one-way ANOVA followed by the Fisher's LSD test for multiple mean comparisons. *P<0.05, **P<0.01 and ***P<0.001 *vs.* Sham; ^{#}P<0.05, ^{##}P<0.01 and ^{###}P<0.001 vs. untreated OVA group; ^{ϕ}P<0.05 *vs.* the respective Bud equimolar dose.

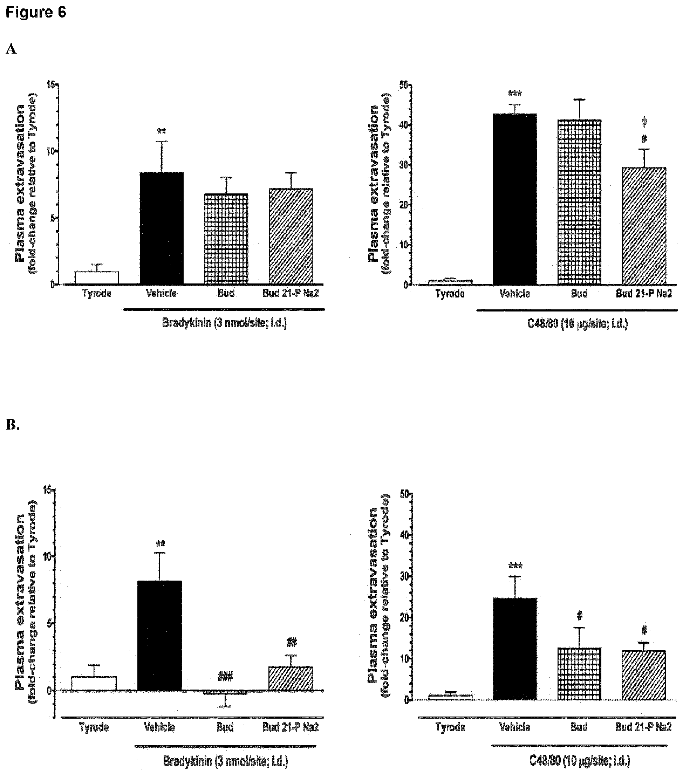

**Figure 6** shows the comparative effects of budesonide and its 21-phosphate sodium salt on plasma extravasation assessed by the Evans blue method, 30 min after the i.d. injection of the edema inducers. The animals were pre-treated (i.p.) with equimolar doses of the corticosteroids, 60 min before edema induction. **Panel A** shows the results obtained after treatment with 2.32µmol/kg of each of the test compounds (equivalent to 1.0 mg/kg Bud and 1.3 mg/kg Bud-21 P-Na₂). **Panel B** shows the results obtained after treatment with 0.70 µmol/kg of each of the test compounds (equivalent to 0.30 mg/kg Bud and 0.39 mg/kg Bud-21 P-Na₂). **P<0.01 and ***P<0.001 *vs.* Tyrode; ^{#}P<0.05 and ^{##}P<0.01 vs. Vehicle-treated group, as analyzed by one-way ANOVA followed by the Fisher's test. ^{ϕ}P<0.05 vs. the respective Bud equimolar dose.

### DETAILED DESCRIPTION OF THE INVENTION

As it will be disclosed in detail in the Experimental Section, the present inventors have found that Bud-21 P-Na₂ is more potent than parent budesonide to exert its beneficial effects in mice with OVA-induced allergic asthma and in cutaneous induced edema.

Furthermore, the present inventors have also found that the effect of Bud-21 P-Na₂ is dose-related and significantly present already in the first hours of the acute first phase of paw edema.

Accordingly, a first object of the present invention is Bud-21 P-Na₂ for use as anti-inflammatory agent or as anti-asthmatic agent.

The second object of the present invention is a pharmaceutical composition comprising Bud-21 P-Na₂ in combination with at least one physiologically acceptable excipient, for use as anti-inflammatory agent or as anti-asthmatic agent.

Bud-21 P-Na₂ can be administered as the sole active principles or in combination with other therapeutically active compounds, preferably antibiotics.

According to one preferred embodiment, said antibiotic is selected from the group comprising gentamicin, tobramycin, tetracycline, neomycin, bacitracin, nystatin, levofloxacin, ciprofloxacin, moxifloxacin, vancomycin, teicoplanin, amikacin, linezolid, and fosfomycin.

Preferably, said compositions are in the form of powder, suspension, or solution, more preferably said compositions are administered by inhalation, parenteral route (intramuscular, intradermal, subcutaneous, intravenous, intra-arterial, intrathecal, intrasynovial), enteral route (oral, sublingual and rectal), cutaneous route, transdermal route, auricular (otic) route, or intraocular route.

The compositions may be used for inhalation through mucosa or consists in a solution for aerosol therapy. For the administration by inhalation, the compound of the invention may be delivered in the form of an aerosol spray in pressurized packs or by use of nebulisers. Moreover, the formulation may also be delivered as a powder inhaled through the insufflations of inhaler devices. The preferred delivery system for inhalation is a metered dose inhalation aerosol formulated as a suspension or solution of the ingredients in suitable propellants for inhalable pharmaceutical preparations.

The pharmaceutical compositions suitable for oral use can be administered in the form of tablets, capsules, or syrups.

According to one preferred embodiment, Bud-21 P-Na₂ and its pharmaceutical composition as defined above, are useful in the prevention and/or treatment of acute or chronic inflammatory diseases, preferably respiratory inflammatory pathologies, obstructive pathologies, allergen-induced airway dysfunctions, such as asthma, COPD and pulmonary fibrosis (e.g. cystic fibrosis), skin inflammatory diseases, such as psoriasis, rheumatoid arthritis, lupus, arthralgia, inflammatory neurodegenerative diseases, such as multiple sclerosis, acute or chronic neuritis.

The third object of the present invention is a product or kit for use as defined above comprising: A) Bud-21 P-Na₂ and its pharmaceutical composition as defined above, and B) at least one antibiotic, A) and B) being two separate formulations for simultaneous, separate or sequential use.

Another object of the present invention relates to the use of Bud-21 P-Na₂ or its pharmaceutical composition as defined above, for the preparation of an anti-inflammatory agent or an anti-asthmatic agent.

According to one preferred embodiment, the present invention relates to the use of Bud-21 P-Na₂ or its pharmaceutical composition as defined above for the preparation of a medicament for the prevention and/or treatment of acute or chronic inflammatory diseases, preferably respiratory inflammatory pathologies, obstructive pathologies, allergen-induced airway dysfunctions, such as asthma, COPD and pulmonary fibrosis (e.g. cystic fibrosis), skin inflammatory diseases, such as psoriasis, rheumatoid arthritis, lupus, arthralgia, inflammatory neurodegenerative diseases, such as multiple sclerosis, acute or chronic neuritis.

Another object of the present invention relates to a method for the prevention and/or treatment of acute or chronic inflammatory diseases, preferably respiratory inflammatory pathologies, obstructive pathologies, allergen-induced airway dysfunctions, such as asthma, COPD and pulmonary fibrosis (e.g. cystic fibrosis), skin inflammatory diseases, such as psoriasis, rheumatoid arthritis, lupus, arthralgia, inflammatory neurodegenerative diseases, such as multiple sclerosis, acute or chronic neuritis comprising the step of administering to a subject in need there of a therapeutically effective amount of Bud-21 P-Na₂ or a pharmaceutical composition thereof.

The invention will be further illustrated in greater details in the following experimental section.

### EXPERIMENTAL SECTION

### Materials and Methods

### a) Chemistry

### 1. Materials and Methods

All the other commercial products have been purchased from Merck Life Science. ¹H (500 MHz) and ¹³C (125 MHz) NMR spectra were recorded on an Agilent INOVA spectrometer; chemical shifts were referenced to the residual solvent signal (CD₃OD: δ_{H} = 3.31, δ_{C} = 49.0). Homonuclear ¹H connectivities were determined by COSY experiments. Two and three bond ¹H-¹³C connectivities were determined by gradient 2D HMBC experiments optimized for a ^{2,3}J of 8 Hz. X-ray powder diffraction (XRPD) was performed using a Panalytical X'pert PRO diffractometer. Intensity profiles were collected in the 2θ range of 4-40° using Ni-filtered CuKα radiation (λ = 1.5406 Å) at 40 kV and 30 mA, with a step size 0.02°, at a scanning time of 120 s/step. The diffraction patterns were processed using the Highscore Plus suite. IR spectra were recorded on Thermo Nicolet 5700 FT-IR spectrometer.

### 2. Synthesis of Budesonide 21-phosphate sodium salt (II)

### Step 1: Synthesis of Budesonide 21-phosphate

To a stirred solution of budesonide (10 g, 0.023 mol) in anhydrous THF (35 mL) at -40 °C was added diphosphoryl chloride (8.0 mL, 0.058 mol), and the resulting mixture was stirred at -40 °C for 20 min. The reaction was quenched with water and treated with saturated sodium bicarbonate solution until pH ~ 8 keeping the mixture under stirring for an hour at room temperature. The solution was extracted with ethyl acetate; the aqueous phase was made acidic using a 1 N HCl solution and extracted several times with ethyl acetate. The combined organic phases were washed with brine, dried over sodium sulfate, and concentrated to give budesonide 21-phosphate (10.1 g, 86%). M.P. 219-221 °C LRMS (ES) (M + H)⁺: calcd, 510.5; found, 511.2.

¹H NMR (500 MHz, CD₃OD) δ 7.45 (d, J = 10.1 Hz, 1H), 6.25 (d, J = 10.1 Hz, 1H), 6.01 (s, 1H), 5.21 (t, J = 4.9 Hz, 0.5H), 5.14 (d, J = 7.2 Hz, 0.5H), 5.01 - 4.83 (m, 2H), 4.77 - 4.59 (m, 2H), 4.47 - 4.37 (m, 1H), 2.65 (td, J = 13.4, 5.3 Hz, 1H), 2.37 (d, J = 9.6 Hz, 1H), 2.28 - 2.07 (m, 3H), 2.01 - 1.92 (m, 1H), 1.87 - 1.79 (m, 1.5H), 1.77 - 1.67 (m, 1.5H), 1.64 - 1.56 (m, 3H), 1.54 - 1.45 (m, 4H), 1.03 - 0.88 (m, 7H).

¹³C NMR (126 MHz, CD₃OD) δ 206.14, 204.88, 190.12, 175.51, 160.99, 133.16, 129.17, 123.87, 110.73, 106.88, 101.11, 100.26, 85.59, 84.37, 71.71, 58.41, 55.43, 52.57, 48.30, 47.18, 42.53, 39.49, 37.38, 36.76, 35.60, 34.27, 33.68, 32.95, 22.83, 19.68, 19.32, 19.21, 18.93, 15.63, 15.55.

### Step 2: Synthesis of Budesonide 21-phosphate sodium salt

Budesonide 21-phosphate (5.0 g, 9.79 mmol) was suspended in water (100 mL) and titrated with 2N NaOH to pH 7.94, obtaining a completely clear solution. Then the solvent was removed, and the residue was treated with methanol (75 mL) keeping the suspension at the boiling point of the solvent for 30 min. After cooling, the insoluble solid was filtered off and the solvent was removed *in vacuo.* The residue was then treated with diethyl ether affording Bud-21 P-Na₂ as a white solid (4.3 g, 79%), M.P. 245-246 °C.

¹H NMR (500 MHz, CD₃OD) δ 7.47 (d, J = 10.1 Hz, 1H), 6.26 (d, J = 10.1 Hz, 1H), 6.01 (s, 1H), 5.18 (dd, J = 13.1, 6.2 Hz, 1H), 4.96 - 4.83 (m, 2H), 4.72-4.62 (m, 2H), 4.41 (d, J = 3.5 Hz, 1H), 2.64 (dt, J = 13.0, 6.7 Hz, 1H), 2.37 (d, J = 11.0 Hz, 1H), 2.24 - 2.09 (m, 3H), 1.94 (dd, J = 17.8, 9.7 Hz, 1H), 1.70 (dd, J = 14.1, 6.6 Hz, 1H), 1.60 (dd, J = 12.1, 7.0 Hz, 3H), 1.51 - 1.39 (m, 4H), 1.04 - 0.89 (m, 7H).

¹³C NMR (CD₃OD-d4): δ 210.89, 209.55, 188.85, 174.28, 159.86, 127.84, 122.55, 109.41, 105.45, 99.88, 98.97, 84.01, 82.92, 70.53, 70.48, 69.96, 69.68, 57.17, 57.08, 54.22, 51.33, 47.07, 45.98, 45.94, 41.34, 40.97, 38.27, 36.17, 35.50, 35.35, 34.34, 33.83, 33.01, 32.47, 31.75, 21.55, 18.44, 17.98, 17.82, 17.54, 14.40, 14.26.

### b) Pharmacology

### 1. Animals

### 1.1. Protocol-1

Female BALB/c mice and male CD-1 were purchased by Charles River (Lecco, Italy). The animals were housed in a controlled environment and provided with standard rodent chow and water. All the animals were anaesthetized before being humanely sacrificed by carbon dioxide euthanasia. All efforts were made to minimize the number of animals used and their suffering. Mice were blindly randomized and experimental procedures and protocols followed national (Direttiva 2010/63/UE) laws and policies and approved by the Italian Ministry of Health.

### 1.2. Protocol-2

Male Balb/c SPF mice (26±2 g, 6-week-old) were purchased from the animal house facilities (CEMIB) at the State University of Campinas (UNICAMP, Brazil). They were group-housed in a temperature-controlled room at 22°C with a 12/12-hour light/dark cycle and allowed free access to food and water.

### 1.3. Protocol-3

Male C57BI/6 SPF mice (25±2 g, 6-week-old) were purchased from the animal house facilities at the São Paulo School of Medicine (Federal University of São Paulo, Brazil). They were group-housed in a temperature-controlled room at 22°C with a 12/12-hour light/dark cycle, had free access to food and water and were allowed to acclimate to our local facility for one week before the start of the experimental procedures. The study agrees with the Ethical Principles for Animal Research established by the Brazilian College for Animal Experimentation (COBEA). According to the internal laboratory rules, euthanasia is performed if severe distress related to the test agents developed during the experiment.

### 2. Test substances and reagents

In Protocol 1 (paw edema), the test compounds budesonide (Bud) and budesonide 21-phosphate sodium salt (Bud 21-P Na₂) have been tested. Both compounds were administered in a volume of 100µl intraperitoneally 1 hour before the induction of the inflammatory reaction. Doses of 0.1, 0.3 e 1mg/kg were tested.

In air pouch induced by ovalbumin in sensitized mice, Bud and Bud 21-P Na₂ (0.3mg/kg) were tested. Both compounds were administered in a final volume of 100µl within the air pocket 1h before the induction of the inflammatory reaction.

In Protocol 2, the test compounds Bud (MW: 430.53 g/mol) and Bud-21 P Na₂ (MW: 554.48 g/mol) were administered at equimolar doses of 3, 10 and 30 nmol/animal/day, equivalent to 1.3, 4.3 and 12.9 µg/animal/day of Bud, 1.7, 5.5 and 16.6 µg/animal/day of Bud-21 P Na₂, respectively. The compounds were dissolved in 10% sterile saline + 90% DMSO (Sigma Chemical Co., St. Louis, MO) at the correspondent concentrations at which the above-mentioned doses per animal result from the intra-nasal (i.n.) administration of 10 µl/animal (5 µl/nostril) of each of the solutions.

Chicken egg-white ovalbumin (OVA; grade V, cat. A5503, Sigma Chemical Co., St. Louis, MO) was dissolved in sterile phosphate buffered saline (PBS) solution (250 µg/ml) and Al(OH)₃ was added (at 13 mg/ml). This mix was used to induce the allergic sensitization of the animals by subcutaneous injection. OVA was dissolved at 1% in sterile PBS solution and this solution was nebulized (as the immunological challenges).

In Protocol 3, Bud (at doses of 1.0 and 0.30 mg/kg) and Bud 21-P Na₂ (at doses of 1.3 and 0.39 mg/kg) were administered on a molar base, these doses were equivalent to 0.70 and 2.32 µmol/kg of each compound, respectively. The vehicle for dissolution of the compounds was sterile saline solution (0.9% NaCl) containing 12.5% DMSO (Sigma Chemical Co., St. Louis, MO). The compound solutions were prepared at concentrations such that the administered dose per kg of body weight results from the intraperitoneal (i.p.) administration of 10 ml/kg of each of the solutions.

Bradykinin acetate (BK; cat. B3259, Sigma Chemical Co., St. Louis, MO) and compound 48/80 (C48/80; cat. C2313, Sigma Chemical Co., St. Louis, MO) were respectively dissolved in sterile Tyrode solution at concentrations of 60 µM and 200 µg/ml (in this way, the i.d. injection of 50 µl of each of the agents resulted in doses of 3 nmol and 10 µg per site of injection, respectively).

### 3. Experimental Groups I

### 3.1 Paw edema

Carrageenan edema in the paw of the CD-1 mice involves the sub-plantar administration of 50µl of 1% carrageenan and the measurement of the increase in the volume of the paw by using a hydroplethysmometer. This experimental model has a biphasic development (Posadas et al., Br J Pharmacol 2004 May;142(2):331-338). The first phase, which is acute, is sustained by an increase in permeability accompanied by infiltration of neutrophils, which develops and resolves between 4 and 5 hours. The second phase occurs after 24 hours and is characterized by macrophage infiltration and has the characteristics of sub chronic inflammation, which reaches its maximum after 72 hours and resolves after 5 days. Bud and Bud 21-P Na₂ have been tested. Both compounds were administered in a volume of 100µl intraperitoneally 1 hour before the induction of the inflammatory reaction. Doses of 0.1, 0.3 e 1mg/kg were tested.

### 3.2 Air Pouch

This model is used to investigate the mechanisms underlying the action of anti-inflammatory drugs. The experimental model involves the subcutaneous injection of air on the back of the animal with the formation of an air pocket which creates an artificial cavity. In this cavity, inflammatory agents can be inoculated allowing to easily collect the cell infiltrate to be analyzed. The formation of the air pocket is obtained through the administration of 5ml of air repeated three days later in mice sensitized with ovalbumin (OVA). The injection of 10 µg of OVA into the dorsal air pouch causes cell accumulation as early as 6 hours with a peak at 24 h and which persists for up to 48 hours. At 24 hours, the cell infiltrate is mainly constituted by the polymorphonuclear cells (PMN) including a significant proportion of eosinophils (Anuk M Das et al, Br J Pharmacol. 1997 May; 121(1): 97-104). Bud and Bud 21-P Na₂ (0.3mg/kg) were tested. Both compounds were administered in a final volume of 100µl within the air pocket 1h before the induction of the inflammatory reaction.

### 4. Experimental Groups II

The table below shows the groups for evaluation:

| **Group** | **Treatment** | **Dose** | **n** |
|---|---|---|---|
| Sham | DMSO/saline | 10 µl/animal, i.n. | 5 |
| OVA | DMSO/saline | 10 µl/animal, i.n. | 6 |
| OVA + Bud 3 | Bud | 3 nmol/animal/day, i.n. | 6 |
| OVA + Bud 10 | Bud | 10 nmol/animal/day, i.n. | 6 |
| OVA + Bud 30 | Bud | 30 nmol/animal/day, i.n. | 6 |
| OVA + Bud-21 P-Na₂ 3 | Bud-21 P-Na₂ | 3 nmol/animal/day, i.n. | 6 |
| OVA + Bud-21 P-Na₂ 10 | Bud-21 P-Na₂ | 10 nmol/animal/day, i.n. | 6 |
| OVA + Bud-21 P-Na₂ 30 | Bud-21 P-Na₂ | 30 nmol/animal/day, i.n. | 6 |

### 4.1 Induction of airway hyperreactivity and treatments

Mice were sensitized with two subcutaneous injections of 0.2 ml of OVA/Al(OH)₃, with an interval of 7 days (Sham animals received the Al(OH)₃ suspension in PBS with no OVA). Seven days after the second sensitization (i.e., 3rd week), the animals were nebulized twice a week with the 1% OVA solution (Sham and untreated OVA groups were nebulized with PBS) during 20 minutes for the next 2 weeks.

Three hours after the challenges (OVA nebulization) and daily during the next 4 weeks (following the first OVA challenge), mice were intra-nasally treated (10 µl/animal as 5 µl/nostril) with the respective compounds/vehicle, and the last dose was administered 30 min before the Penh measurement at the end of the 6th week.

### 4.2 Lung hyperreactivity / Penh function

Airway responsiveness in conscious, spontaneously breathing animals was measured at the end of the 6th week (i.e., after 4-week OVA challenges + treatments), 24 h after the last OVA/PBS challenge via whole body plethysmography (Buxco Europe Ltd, Winchester, UK), as previously described (Santos et al., 2014). Experiments were carried out in a quiet room by an investigator who was unaware of the nature of treatments.

Aerosolized saline (50 µl/mouse during 60 s) and then the muscarinic agonist methacholine (MCh) at increasing concentrations (3.12, 6.25, 12.5 and 25.0 mg/ml in PBS) was nebulized through an inlet of the main chamber for 3 min each to induce bronchoconstriction and readings were taken and averaged for 6 min following each nebulization. After 20 min, the baseline values usually returned at the end of this period. Enhanced pause (Penh) was measured as an indicator of bronchoconstriction and consequent increase of airway resistance as: *Penh=[(expiration time*/*relaxation time)-1]*/*(maximum expiration flow*/*maximum inspiration flow).*

### 4.3 Collection of BAL fluid and blood samples

After Penh evaluation, the mice were anesthetized with inhaled isofluorane (5% v/v in O₂) and blood samples were collected from the descending abdominal aorta. The mice were then euthanized by exsanguination and bronchoalveolar lavage (BAL) was performed by exposing and cannulating the trachea with a polyethylene tube (1 mm outer diameter) connected to a syringe. The lungs were washed by flushing 300 µL of heparin-containing PBS solution (20 Ul/mL). The retrieved BAL lavage aliquots were obtained, and the same procedure was repeated four additional times. The samples were submitted to centrifugation (1000 g for 10 min) and the cell pellet was resuspended in 200 µL of PBS solution. Total cell counts were determined using a Neubauer chamber, and differential counting was carried out in cytospin (Fanem Mod 2400; São Paulo, Brazil) preparations stained with May-Grünwald dye. Leukocytes were classified based on normal morphological criteria.

### 5. Experimental Groups III

The tables below show the experimental groups for evaluation:

### Experiment #1

| **Treatment** | **Dose** | **n** |
|---|---|---|
| Vehicle | 10 ml/kg; i.p. | 5 |
| Bud | 1.0 mg/kg; i.p. | 5 |
| Bud-21 P-Na₂ | 1.3 mg/kg; i.p. | 5 |

### Experiment #2

| **Treatment** | **Dose** | **n** |
|---|---|---|
| Vehicle | 10 ml/kg; i.p. | 5 |
| Bud | 0.30 mg/kg; i.p. | 5 |
| Bud-21 P-Na₂ | 0.39 mg/kg; i.p. | 5 |

### 5.1 Induction of skin edema and treatments

Skin edema was assessed in mice according to the method previously described by Costa et al. (2006), Vasc. Pharmacol; 45: 209-214 and Yshii et al (2009), Toxicon; 53: 42-52. The mice were anesthetized with urethane (25% w/v, 10 ml/kg, i.p.), had their dorsal skin shaved, and thereafter i.p. treated with the vehicle / test compounds. Fifty-five minutes later, a volume of 100 µL of Evans blue dye solution (0.25% in sterile saline) was intravenously (i.v.) injected via the tail vein. Five minutes later, the BK and C48/80 were intradermally (i.d.) injected into the dorsal skin at a fixed volume of 50 µl using a randomized scheme. The same volume of Tyrode solution 50 µl was injected as a control. Thirty minutes later, a 1 ml blood sample was obtained via cardiac puncture and the mice were killed by urethane overdose followed by cervical dislocation. The blood samples were centrifuged at 6,000 g for 4 min to obtain the plasma. The dorsal skin was removed, and the injected sites were punched out using an 8 mm diameter cork borer. A non-injected skin site distant from the injection sites was also punched and taken as a blank. The dye was extracted from each piece of skin and plasma (100 µl) samples using formamide, and the absorbance of the resulting solutions was measured at 620 nm. The plasma extravasation due to each agent (expressed in µl/site) was calculated as the ratio between the absorbance of each respective skin piece, corrected by the blank value and plasma sample solutions, properly adjusted by the dilution factors [Vasc. Pharmacol (2006); 45: 209-214]. To homogenate data due to the variability of different group of animals (inter-group variations), edema data are expressed as the fold-change response relative to the mean Tyrode (control) response of each experiment.

### 6. Statistical analysis

Data are expressed as arithmetic mean ± SEM from *n* individual animals. Statistical analysis of data was carried out using Software GraphPad Prism v5.01. The results were analysed using one-way ANOVA, followed by the Dunnett's multiple comparison test; differences between group means with *P*<0.05 values were considered as significant.

### Results

### 1. Experimental Groups I

### First phase edema

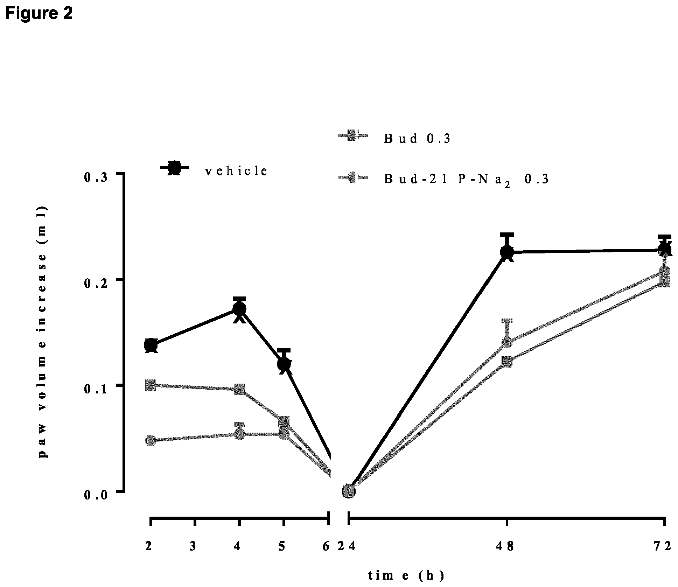

The administration of carrageenan induces in the control animals a significant increase in the volume of the paw already after two hours reaching the maximal response at 4 hours. The results obtained show a difference in terms of pharmacological activity between the two drugs tested in the first phase. The results are summarized as follows:
1. Bud-21 P-Na₂ shows a dose-related anti-inflammatory effect **(****Figure 1****).**
2. Bud-21 P-Na₂ inhibits at a dose of 0.1mg/kg the edema formation by 50% and the maximum inhibition achieved is of 70% with either 0.3 or 1mg/kg. This effect is already at the onset of the edema and last for all the first phase.
3. Bud induces a significant activity profile in inhibiting the edema development. It is to note that the maximum inhibition is about 50% but it is not dose-related **(****Figure 1****).**
4. At a dose of 0.3 kg Bud-21 P-Na₂ shows a more rapid onset of the effect since a significant inhibition is already present after 2 hours and remains constant throughout the first phase. Conversely Bud, at the same dose, reaches the same level of inhibition of Bud-21 P-Na₂ only after 6 hours **(****Figure 2****).**

### Second phase edema

After an apparent resolution of the edema 6 hours after the administration of carrageenan, a second phase develops through a more prolonged and lasting reaction that reaches a plateau after 72 hours. Although the drugs were administered only when the inflammatory reaction was induced, their pharmacological action is also reflected in the second phase. All drugs tested significantly inhibit edema with a similar profile **(****Figure 1****).**

### Air pouch

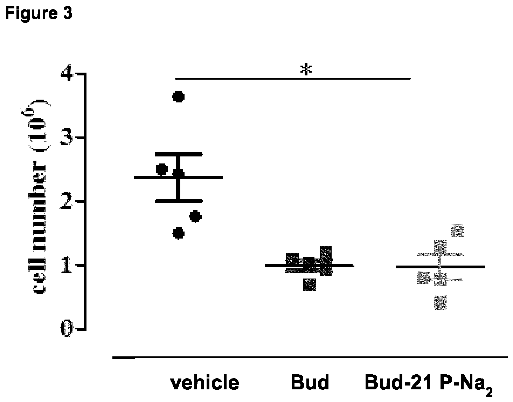

The administration of ovalbumin inside the air pouch of sensitized mice induces a significant cell infiltrate which reaches its maximum after 24 hours. Administration of all compounds significantly inhibit cell recruitment as shown in **Figure 3****.**

### 2. Experimental Groups II

### 2.1 Lung hyperreactivity / Penh function

OVA-induced allergy significantly increased airway responsiveness, as assessed by the Penh measurement following the exposure to increasing concentrations of inhaled methacholine (profiles shown in **Figure 4****,** *Panels A and B*), as evidenced by the increased area under the Penh-methacholine concentration curve (AUC; *Panel* E) and maximal response (Emax; *Panel D).* Basal Penh (i.e., under no methacholine exposure) was not significantly different among the groups.

Both Penh parameters (AUC and Emax) were significantly reduced by Bud-21 P-Na₂ at all the tested doses (3, 10 and 30 nmol/animal/day), while treatment with the parent compound Bud resulted in significant reduction of this response just at the highest dose (30 nmol/animal/day).

### 2.2 BAL fluid cell counts

**Figure 5** shows that the animals with allergic asthma had higher number of total leukocytes recruited to the bronchoalveolar space in comparison with the Sham group, and that all treatments resulted in significant reduction of these numbers, although the response observed in the animals treated with Bud at the 3 nmol/animal/day dose was still significantly higher than that observed in the Sham mice *(panel A).* A more pronounced eosinophil migration to the lungs was observed in the allergic untreated animals, and all the treatments (except for Bud at 3 nmol/animal/day) significantly reduced this cell recruitment *(panel B).* Macrophage recruitment to the bronchoalveolar space was also augmented in the allergic untreated animals, and this response was significantly reduced by all the treatments, although the responses of the animals treated with Bud at either 3 or 10 nmol/animal/day were still significantly higher than those observed in the Sham group *(panel E).* Untreated allergic animals showed no significant neutrophil *(panel C*) or lymphocyte *(panel D)* increase in the BAL fluids, although some of the treatments were capable of diminishing, or even abolishing, the migration of these cells to the bronchoalveolar space.

### 3. Experimental Groups III

### 3.1 Cutaneous edema

As shown in **Figure 6** *(panel A and B),* both BK and C48/80 induced a significant plasma extravasation, measured 30 min after the injection. As shown in panel A, equimolar (2.32 µmol/kg) doses of the two compounds (equivalent to either 1.0 mg/kg Bud and 1.3 mg/kg Bud-21 P-Na₂) administered 60 min before the i.d. injection of the edematogenic agents displayed a similar effect i. e. abolished BK-induced response or reduced, of approximately 50% C48/80-induced plasma extravasation. However, as shown in panel B, when the test compounds were administered at a lower equimolar dose (0.70 µmol/kg equivalent to 0.30 mg/kg Bud or 0.39 mg/kg Bud-21 P-Na₂), Bud was ineffective on either BK or C48/80. Conversely, Bud-21 P-Na₂ significantly inhibited C48/80-induced edema.

### Conclusions

The mouse paw edema model allows to differentiate between an acute immediate phase followed by a sub-chronic response. The finding that the Bud-21 P-Na₂ displays an earlier effect than Bud suggests a feasible faster in onset effect also in future clinical application. Indeed, the effect of the disodium salt is dose-related and significantly present already in the first hours of the acute first phase.

Both drugs were equally active on the second phase of the edema at all the doses tested. This prolonged pharmacological activity is consistent with the known therapeutic profile of this class of anti-inflammatory steroid that involves in the later phase both genomic and non-genomic effect including downregulation of several transcription factors of inflammatory cytokines.

The ability of the drugs to act in a therapeutic frame i.e. in the presence of an established inflammatory state it is confirmed by the experimental study conducted with the air pouch model. In this model, the compounds have shown equal effectiveness in inhibiting the cell recall triggered by the inflammatory stimulus. The lack of difference in this case is because the administration is local and therefore overcomes the PK profile.

Considering the bronchoconstriction response to methacholine (as assessed by the Penh function), the results of Experimental Groups II show that the beneficial therapeutic effects observed with the lowest dose of Bud-21 P-Na₂ (3 nmol/animal/day) were absent when Bud was administered at even higher molar doses (i.e, 3 and 10 nmol/animal/day). A similar situation was observed regarding leukocyte recruitment to the bronchoalveolar space (as assessed by lavage), especially eosinophils (which are the main leukocyte type involved in allergic responses), as well as to macrophages or even total leukocytes.

Considering the cutaneous edema, the results of Experimental Groups III show that Bud was ineffective on either BK or C48/80. Conversely, Bud-21 P-Na₂ significantly inhibited C48/80-induced edema. The results evidence the beneficial effects of pretreatment with the Bud-21 P-Na₂ on mouse skin edema in comparison with the parent compound Bud when administered at an equimolar dose (0.70 µmol/kg) at which the latter is devoid of effects.

In conclusion, these facts clearly evidence that the Bud-21 P-Na₂ is more potent than parent Bud to exert their beneficial effects in mice with OVA-induced allergic asthma and in cutaneous induced edema.

## Claims

1. Budesonide 21-phosphate sodium salt having Formula (II) 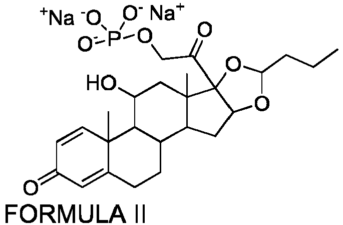 for use as anti-inflammatory agent or as anti-asthmatic agent.

2. A pharmaceutical composition comprising budesonide 21-phosphate sodium salt in combination with at least one physiologically acceptable excipient, for use as anti-inflammatory agent or as anti-asthmatic agent.

3. The pharmaceutical composition for use according to claim 2, further comprising at least one antibiotic, preferably selected from the group comprising gentamicin, tobramycin, tetracycline, neomycin, bacitracin, nystatin, levofloxacin, ciprofloxacin, moxifloxacin, vancomycin, teicoplanin, amikacin, linezolid, and fosfomycin.

4. The pharmaceutical composition for use according to claim 2 or 3, wherein said composition is in the form of powder, suspension, or solution, preferably said composition is suitable for administration by inhalation, parenteral route, enteral route, cutaneous route, transdermal route, auricular (otic) route, or intraocular route.

5. A pharmaceutical composition according to claim 4 wherein said composition is a metered dose inhalation aerosol formulated as a suspension or solution of the ingredients.

6. The budesonide 21-phosphate sodium salt for use according to claim 1 or the pharmaceutical composition for use according to any one of claims 2 to 5 in the prevention and/or treatment of acute or chronic inflammatory diseases, preferably respiratory inflammatory pathologies, obstructive pathologies, allergen-induced airway dysfunctions, such as asthma, COPD and pulmonary fibrosis, skin inflammatory diseases, such as psoriasis, rheumatoid arthritis, lupus, arthralgia, inflammatory neurodegenerative diseases, such as multiple sclerosis, acute or chronic neuritis.

7. A product or a kit for use as anti-inflammatory agent or as anti-asthmatic agent, comprising:
A) budesonide 21-phosphate sodium salt according to claim 1, or a pharmaceutical composition according to claim 2, and
B) at least one antibiotic,
A) and B) being two separate formulations for simultaneous, separate or sequential use.

8. The product or the kit for use according to claim 7 in the prevention and/or treatment of acute or chronic inflammatory diseases, preferably respiratory inflammatory pathologies, obstructive pathologies, allergen-induced airway dysfunctions, such as asthma, COPD and pulmonary fibrosis, skin inflammatory diseases, such as psoriasis, rheumatoid arthritis, lupus, arthralgia, inflammatory neurodegenerative diseases, such as multiple sclerosis, acute or chronic neuritis.

## Patentansprüche

1. Budesonid-21-phosphat-Natriumsalz der Formel (II) 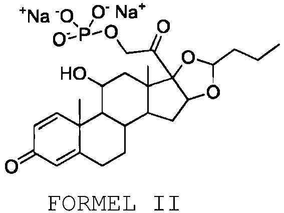 zur Verwendung als entzündungshemmendes Mittel oder als antiasthmatisches Mittel.

2. Pharmazeutische Zusammensetzung, die Budesonid-21-phosphat-Natriumsalz in Kombination mit mindestens einem physiologisch verträglichen Hilfsstoff enthält, zur Verwendung als entzündungshemmendes Mittel oder als antiasthmatisches Mittel.

3. Pharmazeutische Zusammensetzung zur Verwendung gemäß Anspruch 2, die ferner mindestens ein Antibiotikum umfasst, das vorzugsweise aus der Gruppe ausgewählt ist, die aus Gentamicin, Tobramycin, Tetracyclin, Neomycin, Bacitracin, Nystatin, Levofloxacin, Ciprofloxacin, Moxifloxacin, Vancomycin, Teicoplanin, Amikacin, Linezolid und Fosfomycin besteht.

4. Pharmazeutische Zusammensetzung zur Verwendung gemäß Anspruch 2 oder 3, wobei die Zusammensetzung in Form eines Pulvers, einer Suspension oder einer Lösung vorliegt, vorzugsweise die Zusammensetzung zur Verabreichung durch Inhalation, parenterale Verabreichung, enterale Verabreichung, kutane Verabreichung, transdermale Verabreichung, aurikuläre (otische) Verabreichung oder intraokulare Verabreichung geeignet ist.

5. Pharmazeutische Zusammensetzung gemäß Anspruch 4, wobei die Zusammensetzung ein Dosieraerosol ist, das als Suspension oder Lösung der Inhaltsstoffe formuliert ist.

6. Budesonid-21-phosphat-Natriumsalz zur Verwendung gemäß Anspruch 1 oder die pharmazeutische Zusammensetzung zur Verwendung gemäß einem der Ansprüche 2 bis 5 zur Vorbeugung und/oder Behandlung von akuten oder chronischen Entzündungskrankheiten, vorzugsweise Entzündungskrankheiten der Atemwege, obstruktive Erkrankungen, allergeninduzierte Atemwegsfunktionsstörungen wie Asthma, COPD und Lungenfibrose, entzündliche Hauterkrankungen wie Psoriasis, rheumatoide Arthritis, Lupus, Arthralgie, entzündliche neurodegenerative Erkrankungen wie Multiple Sklerose, akute oder chronische Neuritis.

7. Produkt oder Kit zur Verwendung als entzündungshemmendes Mittel oder als antiasthmatisches Mittel, welches Folgendes umfasst:
A) Budesonid-21-phosphat-Natriumsalz gemäß Anspruch 1 oder eine pharmazeutische Zusammensetzung gemäß Anspruch 2 und
B) mindestens ein Antibiotikum,
A) und B) zwei separate Formulierungen zur gleichzeitigen, getrennten oder aufeinanderfolgenden Verwendung sind.

8. Produkt oder Kit zur Verwendung gemäß Anspruch 7 zur Vorbeugung und/oder Behandlung von akuten oder chronischen Entzündungskrankheiten, vorzugsweise Entzündungskrankheiten der Atemwege, obstruktive Erkrankungen, allergeninduzierte Atemwegsfunktionsstörungen wie Asthma, COPD und Lungenfibrose, entzündliche Hauterkrankungen wie Psoriasis, rheumatoide Arthritis, Lupus, Arthralgie, entzündliche neurodegenerative Erkrankungen wie Multiple Sklerose, akute oder chronische Neuritis.

## Revendications

1. Sel de sodium de budésonide 21-phosphate de formule (II) 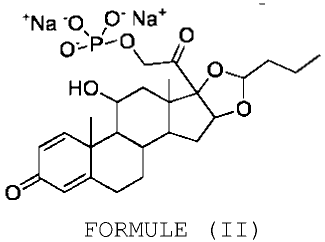 pour une utilisation comme agent anti-inflammatoire ou comme agent antiasthmatique.

2. Composition pharmaceutique comprenant un sel de sodium de budésonide 21-phosphate en combinaison avec au moins un excipient physiologiquement acceptable, pour une utilisation comme agent anti-inflammatoire ou comme agent antiasthmatique.

3. Composition pharmaceutique pour une utilisation selon la revendication 2, comprenant en outre au moins un antibiotique, de préférence choisi dans le groupe comprenant la gentamicine, la tobramycine, la tétracycline, la néomycine, la bacitracine, la nystatine, la lévofloxacine, la ciprofloxacine, la moxifloxacine, la vancomycine, la teicoplanine, l'amikacine, le linézolide et la fosfomycine.

4. Composition pharmaceutique pour une utilisation selon la revendication 2 ou 3, dans laquelle ladite composition est sous forme de poudre, de suspension ou de solution, de préférence ladite composition est adaptée à une administration par inhalation, voie parentérale, voie entérale, voie cutanée, voie transdermique, voie auriculaire (otique) ou voie intraoculaire.

5. Composition pharmaceutique selon la revendication 4, dans laquelle ladite composition est un aérosol d'inhalation à dose mesurée formulé sous forme de suspension ou de solution des ingrédients.

6. Sel de sodium de budésonide 21-phosphate pour une utilisation selon la revendication 1 ou composition pharmaceutique pour une utilisation selon l'une quelconque des revendications 2 à 5 dans la prévention et/ou le traitement de maladies inflammatoires aiguës ou chroniques, de préférence de pathologies inflammatoires respiratoires, de pathologies obstructives, de dysfonctionnements des voies respiratoires induits par des allergènes, tels que l'asthme, la BPCO et la fibrose pulmonaire, de maladies inflammatoires cutanées, telles que le psoriasis, la polyarthrite rhumatoïde, le lupus, l'arthralgie, de maladies neurodégénératives inflammatoires, telles que la sclérose en plaques, de névrites aiguës ou chroniques.

7. Produit ou un kit pour une utilisation comme agent anti-inflammatoire ou comme agent antiasthmatique, comprenant :
A) un sel de sodium de budésonide 21-phosphate selon la revendication 1, ou une composition pharmaceutique selon la revendication 2, et
B) au moins un antibiotique,
A) et B) étant deux formulations distinctes pour une utilisation simultanée, séparée ou séquentielle.

8. Produit ou kit pour une utilisation selon la revendication 7 dans la prévention et/ou le traitement de maladies inflammatoires aiguës ou chroniques, de préférence de pathologies inflammatoires respiratoires, de pathologies obstructives, de dysfonctionnements des voies aériennes induits par des allergènes, tels que l'asthme, la BPCO et la fibrose pulmonaire, de maladies inflammatoires cutanées, telles que le psoriasis, la polyarthrite rhumatoïde, le lupus, l'arthralgie, de maladies neurodégénératives inflammatoires, telles que la sclérose en plaques, de névrites aiguës ou chroniques.
